# EUROPÄISCHE PATENTSCHRIFT

(11) **EP 4 511 110 B1**
(45) Veröffentlichungstag und Bekanntmachung des Hinweises auf die Patenterteilung: **23.09.2026**
(21) Anmeldenummer: 23722269.0
(22) Anmeldetag: 20.04.2023
(51) Int. Cl.: A61N 5/06

(54) **DRAHTLOSE MAGNETOELEKTRISCHE LICHTQUELLE**
WIRELESS MAGNETOELECTRIC LIGHT SOURCE
SOURCE DE LUMIÈRE MAGNÉTOÉLECTRIQUE SANS FIL

(30) Priorität: 22.04.2022 DE 102022109762
(43) Veröffentlichungstag der Anmeldung: 26.02.2025
(73) Patentinhaber: Universität zu Köln, 50931 Köln (DE)
(72) Erfinder: GATHER, Malte C., 50678 Köln (DE); BUTSCHER, Julian F., 50931 Köln (DE)
(74) Vertreter: Michalski Hüttermann & Partner mbB
(86) Internationale Anmeldenummer: PCT/EP2023/060336
(87) Internationale Veröffentlichungsnummer: WO 2023/203153

(56) Entgegenhaltungen:
- CN-A- 113 949 168
- US-A1- 2009 062 886

## Beschreibung

Die Erfindung betrifft ein System zum Bereitstellen eines optischen Signales.

Die Erfindung betrifft weiterhin die Verwendung des obigen Systems zur optischen Stimulation einer Nervenzelle.

Die Erfindung betrifft ferner ein Verfahren zur Herstellung des obigen Systems.

Optogenetik ist eine biologische Technologie, die durch genetische Modifikation neuronaler Zellen deren Stimulation mit Hilfe von Licht ermöglicht, wodurch die selektive Erforschung biologischer neuronaler Netzwerke möglich wird. Dadurch lassen sich beispielsweise Erkenntnisse über neurodegenerative Krankheiten wie Parkinson und/oder Alzheimer sammeln.

Während die biotechnologischen Werkzeuge der Optogenetik in den letzten Jahren rasant verbessert wurden, hinkt die Entwicklung adäquater Lichtquellen zur Bereitstellung des optischen Signals für die Zelle noch immer hinterher. Da bei Verwendung eine Implantation der Lichtquellen in das zu untersuchende biologische System vorgesehen sein kann, sollte die Lichtquelle zur Minimierung von Fremdkörperkontakt drahtlos betreibbar und zudem möglichst klein sein, um den Schaden aufgrund der Implantation möglichst gering zu halten.

Bereits aus der DE 100 25 028 A1 bekannt ist der äußerst kompakte Kleinleistungs-Spannungswandler für niederfrequente Impuls- und Wechselspannungen, der mit relativ hohem Wirkungsgrad sowie Übertragungsfaktoren bis zum 10000-fachen eine sichere galvanische Trennung zwischen Ein- und Ausgangsseite bei geringstem Isolationsaufwand auf engstem Raum gewährleistet.

Die WO 2020/ 206 332 A1 beschreibt neue Geräte, Systeme und Verfahren, die magnetoelektrische Nervenstimulatoren mit abstimmbarer Amplitude und Wellenform verwenden. Spezifische Ausführungsformen umfassen einen magnetoelektrischen Film, einen Magnetfeldgenerator und eine elektrische Schaltung, die mit dem magnetoelektrischen Film gekoppelt ist, wobei in bestimmten Ausführungsformen die elektrische Schaltung Komponenten umfasst, die so konfiguriert sind, dass sie ein von dem magnetoelektrischen Film erzeugtes elektrisches Ausgangssignal modifizieren.

Bei dem Zündimpulsgenerator aus der WO 01/ 91 200 A1 handelt es sich um einen Spannungswandler zur Erzeugung von Hochspannungsimpulsen bis 30 kV. Der Zündimpulsgenerator setzt sich aus einem magnetomechanischen Wandler, bestehend aus dem magnetostriktiven Aktorkern und der ihn umgebenden Spule mit Flussleitblech, aus einem Isolator und aus einem elektromechanischen Wandler, der als Piezozündelement ausgeführt ist, zusammen. Durch die gleichzeitige Ausnutzung des magnetostriktiven Effekts und des direkten Piezoeffekts erfolgt die Umwandlung der Spannung. Der Zündimpulsgenerator dient der Entzündung technischer Gase und zum elektrischen Zünden pyrotechnischer Zündsätze.

Aus der US 7 808 236 B1 sind eine Vorrichtung - genannt Harvester - und ein Verfahren zum Sammeln von Energie aus der Umwelt und/oder anderen externen Quellen und deren Umwandlung in nützliche elektrische Energie bekannt. Der Harvester enthält keinen Permanentmagneten oder eine andere lokale Feldquelle, sondern stützt sich auf das Erdmagnetfeld oder eine andere Quelle eines Magnetfeldes, das sich außerhalb der Sensorvorrichtung befindet. Ein Vorteil dieser neuen Harvester besteht darin, dass sie kleiner und leichter gebaut werden können als Energie-Harvester, die einen Magneten und/oder eine Trägheitsmasse enthalten.

Die WO 2010/ 097 407 A1 bezieht sich auf eine Lichtquelle, die einen piezoelektrischen Transformator und eine oder mehrere Vorrichtungen auf Halbleiterbasis umfasst, die Elektrolumineszenz zeigen und elektrisch damit verbunden sind. Der piezoelektrische Transformator ist so konfiguriert, dass eine inhärente elektrische Eigenschaft eine vorbestimmte Obergrenze für einen Ausgangsstrom festlegt, der von einem Ausgangsanschluss des piezoelektrischen Transformators verfügbar ist.

In der DE 10 2006 040 731 A1 wird eine Vorrichtung, insbesondere ein Mikrosystem, beschrieben, die eine Einrichtung zur Energieumwandlung umfasst. Die Einrichtung zur Energieumwandlung weist eine piezoelektrische, mechanische schwingfähige Membranstruktur zur Umwandlung von mechanischer Energie in elektrische Energie und/oder umgekehrt auf, wobei die Membranstruktur in einer Umgebung verkapselt angeordnet ist, die einen vorbestimmten Druck aufweist, der insbesondere geringer als ein isostatischer Druck ist.

Die US 2016 / 0 303 402 A1 umfasst Verfahren und Vorrichtungen zur Modulation der Aktivität oder der Aktivitäten lebender Zellen, wie Zellen, die in Menschen, Tieren, Pflanzen, Insekten, Mikroorganismen und anderen Organismen vorkommen oder von ihnen stammen. Die Verfahren umfassen die Anwendung von Ultraschall, wie z. B. Ultraschall mit niedriger Intensität und niedriger Frequenz, auf lebende Zellen, um die Zellen zu beeinflussen und die Aktivitäten der Zellen zu modulieren.

Die US 2009/062886 A1 betrifft ein kleines implantierbares magnetostriktiv-elektroaktives Gerät zur Abgabe elektrischer Energie an das umliegende Gewebe. Das ME-Gerät ermöglicht die Stimulation eines Nervs, Gewebes oder inneren Organs mit Gleichstrom, beispielsweise mit relativ schwachem Gleichstrom für eine temporäre oder bedarfsgerechte Therapie.

Die CN 1113 949 168 A beschreibt ein in-vivo implantierbares optisches medizinisches Gerät und dessen Herstellungsverfahren. Das in-vivo implantierbare optische medizinische Gerät umfasst: ein flexibles Substrat, eine Leuchtstruktur, eine Lithiumbatterie, eine Ladeinduktionsstruktur und eine Verpackungsschicht.

Ausgehend davon ist es Aufgabe der Erfindung, eine Lichtquelle bereitzustellen, die drahtlos betreibbar ist und sich besonders einfach verkleinern lässt.

Die Lösung der Aufgabe erfolgt erfindungsgemäß durch die Merkmale der unabhängigen Ansprüche. Bevorzugte Ausgestaltungen der Erfindung sind in den Unteransprüchen angegeben, die jeweils einzeln oder in Kombination einen Aspekt der Erfindung darstellen können.

Erfindungsgemäß wird ein System zum Bereitstellen eines optischen Signales umfassend einen Energiewandler und eine Lichtquelle bereitgestellt, wobei der Energiewandler wenigstens zwei Materialien umfasst und als magnetoelektrischer Wandler dazu ausgestaltet ist, auf Basis eines magnetischen Wechselfeldes elektrische Energie zu erzeugen, wobei die Lichtquelle auf dem Energiewandler angeordnet ist und eine erste als Elektrode fungierende Oberfläche des Energiewandlers elektrisch leitend kontaktiert und wobei die Lichtquelle nicht über eine Kabelverbindung mit dem Energiewandler elektrisch kontaktiert ist.

Ein Aspekt der Erfindung ist, dass die Energieversorgung der Lichtquelle über den Energiewandler bereitgestellt wird, wobei der Energiewandler als magnetoelektrischer Wandler dazu ausgestaltet ist, auf Basis eines magnetischen Wechselfeldes elektrische Energie zu erzeugen. Dies ermöglicht die Lichtquelle drahtlos zu betreiben.

Ein weiterer Aspekt der Erfindung ist, dass die Lichtquelle auf dem Energiewandler angeordnet ist. Dabei ist vorgesehen, dass zwischen der Lichtquelle und der ersten als Elektrode fungierenden Oberfläche des Energiewandlers eine elektrisch leitende Verbindung vorhanden ist. Die Lichtquelle kann berührend auf der ersten Oberfläche angeordnet sein und diese flächig kontaktieren. Alternativ kann die Lichtquelle auf dem Energiewandler angeordnet sein, die erste Oberfläche aber nicht flächig kontaktieren, sondern lediglich über eine oder mehrere Schichten elektrisch leitend mit der ersten Oberfläche verbunden sein. In beiden Fällen dient der Energiewandler als Substrat für die Lichtquelle. Derart wird ermöglicht, dass auf Kabelverbindungen als elektrische Verbindung zwischen dem Energiewandler und der Lichtquelle verzichtet werden kann. Entsprechend kann das System besonders klein ausgestaltet werden und lässt sich zudem besonders gut miniaturisieren.

In diesem Zusammenhang ist zudem vorgesehen, dass die Lichtquelle nicht über eine Kabelverbindung mit dem Energiewandler elektrisch kontaktiert ist. Somit weist das System also keine Kabelverbindung als elektrischer Leiter zwischen der Lichtquelle und dem Energiewandler auf. Derart ist das System besonders klein und robust. Dies macht das System besonders geeignet, um in ein zu untersuchendes biologisches System implantierbar zu sein.

In Zusammenhang mit dem Energiewandler ist gemäß einer bevorzugten Weiterbildung vorgesehen, dass eines der wenigstens zwei Materialien als piezoelektrisches Material ausgestaltet ist und das andere der wenigstens zwei Materialien als magnetostriktives Material ausgestaltet ist.

Ein piezoelektrisches Material ist ein Material, bei dem Piezoelektrizität, auch piezoelektrischer Effekt genannt, auftritt - sprich das Auftreten einer elektrischen Spannung, wenn das Material elastisch verformt wird. Ein magnetostriktives Material ist ein Material, beim dem infolge eines angelegten magnetischen Feldes eine Deformation eintritt. Der Energiewandler basiert also bevorzugt auf dem Komposit-Magnetoelektrischen-Effekt, bei dem die Eigenschaften magnetostriktiver und piezoelektrischer Materialien durch mechanische Kopplung miteinander verbunden sind. Im Magnetfeld verformt sich das magnetostriktive Material, was zu einer elastischen Verformung des piezoelektrischen Materials führt, wodurch eine elektrische Spannung erzeugt wird. Die Ausgestaltung des Energiewandlers in dieser Form hat den Vorteil, dass trotz der geringen Größe des Energiewandlers zur Erzeugung von elektrischer Energie ein niederfrequentes magnetisches Wechselfeld verwendet werden kann, das von biologischem Gewebe kaum abgeschwächt wird. Entsprechend kann die Lichtquelle auch in von biologischem Gewebe umgebenen Zustand ohne signifikante Energieverluste drahtlos betrieben werden. Ein weiterer Vorteil liegt darin, dass sich der Energiewandler besonders gut miniaturisieren lässt.

Das piezoelektrische Material ist besonders bevorzugt ausgewählt aus der Gruppe umfassend piezoelektrische Keramiken, piezoelektrische Kristalle und/oder piezoelektrische Kunststoffe und insbesondere Blei-Zirkonat-Titanat (PZT), Blei-Magnesium-Niobat-Blei-Titanat (PMN-PT) und/oder Polyvinylidenfluorid (PVDF). Diese Materialien haben sich also besonders geeignet für den Energiewandler erwiesen.

Das magnetostriktive Material ist besonders bevorzugt ausgewählt aus der Gruppe umfassend metallische Gläser und/oder amorphe Metalle und insbesondere, Eisen-Silizium-Borit (FeSiB), Eisen-Kobalt-Silizium Borit (FeCoSiB) und/oder Nickel. Diese Materialien haben sich also besonders geeignet für den Energiewandler erwiesen. Weiter bevorzugt ist das piezoelektrische Material PZT und das magnetostriktive Material FeSiB.

In Bezug zur Lichtquelle ist bevorzugt vorgesehen, dass die Lichtquelle mehrere organische Leuchtdioden umfasst. Eine organische Leuchtdiode (englisch organic light-emitting diode, OLED) ist ein leuchtendes Dünnschichtbauelement aus organischen halbleitenden Materialien. Bevorzugt umfasst die OLED zudem zwei elektrisch leitfähige Lichtquellenelektroden. OLEDs haben den Vorteil, dass sie sich in Dünnschichttechnik kostengünstiger herstellen lassen und derart besonders einfach auf den Energiewandler aufgebracht werden können. In anderen Worten sind also bevorzugt eine Mehrzahl an OLEDs, sprich wenigstens zwei OLEDs, auf dem Energiewandler aufgebracht. Weiter bevorzugt ist vorgesehen, dass die OLEDs dazu ausgestaltet sind, ein optisches Signal im sichtbaren und/oder infraroten Bereich, bevorzugt zwischen 380 nm und 1000 nm zu erzeugen. Dies macht das optische Stimulieren von Nervenzellen besonders einfach.

Gemäß einer bevorzugten Weiterbildung der Erfindung ist vorgesehen, dass die Lichtquelle zwei antiparallel geschaltete OLEDs umfasst. Antiparallel geschaltet bedeutet, dass bei einer Parallelschaltung der OLEDs, bei der zwei Strompfade gebildet werden, eine der zwei OLEDs in Sperrrichtung und die andere der zwei OLEDs in Durchlassrichtung verschaltet ist. Mittels dieser elektrischen Verschaltung zweier OLEDs lässt sich trotz der kapazitiven Eigenschaften des Energiewandlers, der sich nicht wie eine ideale Wechselspannungsquelle, sondern sich im Grunde wie ein sich periodisch ladender und entladender Kondensator verhält, ein optisches Signal erzeugen. Dies hat weiterhin den Vorteil, dass bei Verwendung des Systems sowohl während des positiven als auch während des negativen sinusoidalen Spannungsverlaufs des Energiewandlers ein optisches Signal erzeugt wird. Aufgrund der üblicherweise verwendeten Betriebsfrequenzen des Energiewandlers, erscheint die Lichtquelle für das menschliche Auge zudem als durchgehend leuchtend. Die zwei OLEDs können gleichartig ausgestaltet sein, also ein optisches Signal mit gleicher Frequenz erzeugen. Alternativ können die zwei OLEDs unterschiedlich ausgestaltet sein und jeweils ein optisches Signal mit zueinander verschiedenen Frequenzen erzeugen.

Grundsätzlich können die zwei OLEDs nebeneinander angeordnet sein. Gemäß einer weiteren bevorzugten Weiterbildung der Erfindung ist allerdings vorgesehen, dass die Lichtquelle zwei übereinandergestapelte OLEDs umfasst. Dies ist eine besonders einfach herstellbare und platzsparende Möglichkeit die Lichtquelle auf dem Energiewandler aufzubringen. Insbesondere bedeutet übereinandergestapelt, dass eine der zwei OLEDs direkt auf dem Energiewandler und insbesondere direkt auf der ersten als Elektrode fungierenden Oberfläche des Energiewandlers aufgebracht ist und die andere der zwei OLEDs auf der ersten OLED aufgebracht ist und derart eine Stapelanordnung erzielt wird. Dies hat den Vorteil, dass die zwei OLEDs bei der Herstellung nacheinander mittels Beschichtungstechniken abgeschieden werden können, so dass die Herstellung des Systems besonders einfach und robust ist.

Weiter bevorzugt kann auch vorgesehen sein, dass die Lichtquelle eine Vielzahl an OLEDs umfasst und insbesondere mehr als zwei OLEDs umfasst. Bevorzugt ist vorgesehen, dass wenigstens zwei der Vielzahl an OLEDs antiparallel zueinander geschaltete sind. Die Orientierung der Schaltung der weiteren OLEDs kann frei gewählt werden. Weiter bevorzugt ist vorgesehen, dass die Vielzahl an OLEDs in einem Stapel oder in mehreren Stapeln übereinadergestapelt sind. Mit einer Vielzahl an OLEDs lassen sich beliebige Farbmischungen wie zum Beispiel weißes Licht sowie eine örtliche Trennung der Lichtquellen erreichen.

Wie bereits erwähnt kontaktiert die Lichtquelle die erste als Elektrode fungierende Oberfläche des Energiewandlers elektrisch leitend. Dabei kann vorgesehen sein, dass zwischen der Lichtquelle und der ersten Oberfläche des Energiewandlers weitere Schichten des Systems angeordnet sind. Alternativ und bevorzugt ist vorgesehen, dass die Lichtquelle direkt auf der ersten Oberfläche des Energiewandlers aufgebracht ist und diese berührt und derart eine elektrisch leitende Verbindung zwischen der ersten Oberfläche und der Lichtquelle vorhanden ist.

Weiter bevorzugt umfasst der Energiewandler zwei einander gegenüberliegende Oberflächen, die beide als Elektroden fungieren. Weiter bevorzugt ist der Energiewandler als magnetoelektrischer Wandler dazu ausgestaltet, auf Basis des magnetischen Wechselfeldes eine elektrische Wechselspannung über seine beiden gegenüberliegenden Oberflächen zu erzeugen.

Zudem sind die erste und/oder die zweite der zwei Oberflächen des Energiewandlers bevorzugt teilweise isoliert. In anderen Worten ist also bevorzugt vorgesehen, dass die Lichtquelle auf der ersten der beiden teilweise isolierten als Elektrode fungierenden Oberfläche des Energiewandlers aufgebracht ist und bevorzugt die erste Oberfläche berührend kontaktiert, derart dass eine elektrisch leitende Verbindung zwischen der ersten als Elektrode fungierenden Oberfläche des Energiewandlers und der Lichtquelle vorhanden ist.

Weiter bevorzugt umfasst die Lichtquelle zwei Lichtquellenelektroden, wobei eine der zwei Lichtquellenelektroden mit der als Elektrode fungierenden ersten Oberfläche des Energiewandlers kontaktiert ist. In diesem Zusammenhang ist gemäß einer weiteren bevorzugten Weiterbildung zudem vorgesehen, dass die Lichtquelle über eine leitende Schicht elektrisch leitendend mit der zweiten als Elektrode fungierenden Oberfläche des Energiewandlers elektrisch kontaktiert ist und eine Isolationsschicht die leitende Schicht von der ersten Oberfläche elektrisch isoliert. In anderen Worten wird also bevorzugt die zweite Lichtquellenelektrode durch die Isolationsschicht von der ersten Oberfläche des Energiewandlers elektrisch isoliert. Somit dient also der partiell isolierte Energiewandler als Substrat für die Lichtquelle, welche über die erste der beiden als Elektrode fungierende Oberfläche des Energiewandlers und über die teilweise Isolation dieser Oberfläche hinweg aufgebracht ist. Die zweite Lichtquellenelektrode wird bevorzugt über die leitende Schicht - bevorzugt eine leitfähige Dünnschichtverbindung - mit der gegenüberliegenden, zweiten als Elektrode fungierenden Oberfläche des Energiewandlers verbunden, sodass der elektrische Stromkreis geschlossen ist.

In Bezug zur Isolationsschicht ist weiter bevorzugt vorgesehen, dass diese alle Oberflächen des Energiewandlers, bis auf jeweils einen Teil der ersten und zweiten Oberfläche des Energiewandlers elektrisch isoliert. Auf dem nicht isolierten Teil ist bevorzugt die Lichtquelle kontaktiert. Die Isolationsschicht dient der elektrischen Separierung der beiden Lichtquellenelektroden, sowie der Vermeidung eines elektrischen Kurzschlusses zwischen den beiden als Elektroden fungierenden ersten und zweiten Oberflächen des Energiewandlers. Die Isolationsschicht umfasst bevorzugt ein isolierendes Material ausgewählt aus der Gruppe umfassend organischer Polymere oder metallische Oxide und insbesondere Parylen-C und Aluminium(III)oxid. Diese Materialien haben sich also als besonders geeignet für die elektrische Isolation erwiesen.

Im Hinblick auf den Energiewandler kann der Energiewandler unterschiedlich ausgestaltet sein. Beispielsweise kann das erste Material in Form von kleinen Partikeln, bevorzugt Nanopartikeln im ersten Material vorliegen, wobei die Partikel beispielsweise homogen verteilt sein können. Alternativ kann das erste Material in Form von Säulen oder anderen Strukturen das zweite Material durchziehen. Bevorzugt ist allerdings gemäß einer weiteren Weiterbildung der Erfindung vorgesehen, dass der Energiewandler einen Schichtaufbau mit wenigstens zwei Schichten aufweist, wobei das erste Material und das zweite Material jeweils als eine Schicht des Energiewandlers ausgebildet ist und wobei die Lichtquelle auf der ersten der zwei Schichten aufgebracht ist und diese elektrisch leitend kontaktiert. Ein Schichtaufbau ist besonders einfach herzustellen.

Grundsätzlich kann vorgesehen sein, dass die erste Schicht als Schicht mit piezoelektrischem Material ausgestaltet ist. Alternativ kann vorgesehen sein, dass die erste Schicht als Schicht mit magnetostriktivem Material ausgestaltet ist. In einer weiteren bevorzugten Weiterbildung der Erfindung ist allerdings vorgesehen, dass die erste Schicht als Schicht mit magnetostriktivem Material ausgestaltet ist. In anderen Worten kontaktiert also die Lichtquelle bevorzugt die Schicht mit magnetostriktivem Material des Energiewandlers oder noch anders ausgedrückt ist die Lichtquelle bevorzugt auf der Schicht mit magnetostriktivem Material aufgebracht. Diese Ausgestaltung hat gegenüber dem Aufbringen der Lichtquelle auf der Schicht mit piezoelektrischem Material den Vorteil, dass bei der Herstellung, die Schicht mit magnetostriktivem Material an der Oberfläche glatter ist als die Schicht mit piezoelektrischem Material, so dass die Oberfläche der Schicht mit magnetostriktivem Material vor Aufbringung der Lichtquelle nicht poliert werden muss. Entsprechend vereinfacht sich die Herstellung des Systems.

Gemäß einer weiteren bevorzugten Weiterbildung der Erfindung ist vorgesehen, dass zwischen der ersten Schicht und der zweiten Schicht eine Verbindungsschicht angeordnet ist. In anderen Worten ist bevorzugt vorgesehen, dass sich die erste und die zweite Schicht nicht direkt berühren, sondern dazwischen die Verbindungsschicht vorhanden ist. Die Verbindungsschicht sorgt für die mechanische Kopplung der ersten und zweiten Schicht. Bevorzugt ist die Verbindungsschicht eine Epoxy-Schicht. Derart kann auf einfache Art der Energiewandler durch Verbinden der ersten und zweiten Schicht mittels der Epoxy-Schicht hergestellt werden. Weiter bevorzugt ist vorgesehen, dass die Verbindungschicht direkt berührend an der Schicht mit magnetostriktivem Material und/oder an der Schicht mit piezoelektrischem Material anliegt.

Weiter bevorzugt ist im Zusammenhang mit dem Schichtaufbau des Energiewandlers vorgesehen, dass die Schicht mit magnetostriktivem Material eine homogene Schicht umfassend ein oder mehrere magnetostriktiven Materialien umfasst. Bevorzugt ist eine Schichtdicke der homogenen Schicht umfassend ein oder mehrere magnetostriktive Materialien zwischen 1 µm und 100 µm. Weiter bevorzugt ist vorgesehen, dass die Lichtquelle auf der homogenen Schicht umfassend ein oder mehrere magnetostriktive Materialien aufgebracht ist. Weiter bevorzugt ist ebenfalls vorgesehen, dass die Schicht mit magnetostriktivem Material ausschließlich aus der homogenen Schicht umfassend ein oder mehrere magnetostriktive Materialien besteht.

Zudem ist weiter bevorzugt vorgesehen, dass die Schicht mit piezoelektrischem Material eine homogene Schicht umfassend ein oder mehrere piezoelektrische Materialien umfasst. Weiter bevorzugt ist eine Schichtdicke der homogenen Schicht umfassend ein oder mehrere piezoelektrische Materialien zwischen 1 µm und 500 µm. Grundsätzlich kann auch die Schicht mit piezoelektrischem Material ausschließlich aus der homogenen Schicht umfassend ein oder mehrere piezoelektrische Materialien bestehen. Da einige piezoelektrische Materialien jedoch nicht intrinsisch leitfähig sind, ist allerdings bevorzugt vorgesehen, dass die homogene Schicht umfassend ein oder mehrere piezoelektrische Materialien an ihrer Oberseite und an ihrer Unterseite mit einer leitfähigen Schicht versehen ist. In anderen Worten umfasst bevorzugt die Schicht mit piezoelektrischem Material nebst der homogenen Schicht umfassend ein oder mehrere piezoelektrische Materialien auch zwei leitfähige Schichten. Die leitfähigen Schichten umfassen oder bestehen bevorzugt aus Metall und besonders bevorzugt aus Nickel.

Gemäß einer weiteren bevorzugten Weiterbildung der Erfindung wird ein System bereitgestellt, bei dem die Lichtquelle und der Energiewandler in eine Abkapselung integriert sind. In anderen Worten sind also der Energiewandler und die auf dem Energiewandler aufgebrachte Lichtquelle bevorzugt abgekapselt und/oder in die Abkapselung integriert. Derart kann das System auf einfache Weise vor schädlichen Umgebungseinflüssen wie Sauerstoff und/oder Feuchtigkeit geschützt werden. Weiter bevorzugt ist vorgesehen, dass die Abkapselung aus einem biokompatiblen Material ist. Besonders bevorzugt ist die Abkapselung optisch transparent, weist also für das optische Signal im sichtbaren Bereich eine optische Transmission von wenigstens 80 % auf. Bevorzugt umfasst die Abkapselung Parylen-C kombiniert mit wechselnden Schichten aus Aluminiumoxid und Zirkoniumoxid.

Gemäß einer weiteren bevorzugten Weiterbildung der Erfindung ist vorgesehen, dass das System eine Energiequelle umfasst, wobei die Lichtquelle und die Energiequelle und/oder der Energiewandler und die Energiequelle nicht über eine elektrische Verbindung miteinander verbunden sind. In anderen Worten wird also auf eine Kabelverbindung zwischen der Energiequelle und dem Energiewandler und/oder der Lichtquelle verzichtet. Bei Verwendung des Systems wird die Lichtquelle also drahtlos betrieben.

In diesem Zusammenhang ist nach einer weiteren bevorzugten Weiterbildung der Erfindung vorgesehen, dass die Energiequelle als Spule ausgestaltet ist und/oder dazu ausgestaltet ist ein magnetisches Wechselfeld zu erzeugen. Die Energieübertragung findet also bevorzugt über das magnetische Wechselfeld von der Energiequelle zum Energiewandler statt. Bevorzugt ist die Energiequelle dazu ausgestaltet ein magnetisches Wechselfeld mit einer Frequenz zwischen 50 kHz und 2 MHz zu erzeugen. Dieser Frequenzbereich hat den Vorteil, dass das magnetische Wechselfeld von biologischem Gewebe nur sehr gering absorbiert wird und derart kaum Energieverluste vorhanden sind. Weiter bevorzugt ist vorgesehen, dass die Energiequelle dazu ausgestaltet ist, ein magnetisches Wechselfeld mit einer Frequenz zu erzeugen, die resonant zu einer Schwingungsfrequenz des Energiewandlers ist. In anderen Worten sind also die Energiequelle und der Energiewandler bevorzugt aufeinander abgestimmt.

Weiter bevorzug ist vorgesehen, dass der Energiewandler und die Lichtquelle zur optischen Stimulation von Nervenzellen ausgestaltet sind. Dies bedeutet bevorzugt, dass die Lichtquelle und der Energiewandler zusammen bevorzugt Abmessungen unterhalb von 11 mm in der längsten äußeren Abmessung umfassen. Besonders bevorzugt ist eine längste seitliche Abmessung der Vorrichtung umfassend den Energiewandler und die Lichtquelle weniger als 1 mm, weiter bevorzugt weniger als 800 µm und besonders bevorzugt weniger als 500 µm.

Die Erfindung betrifft weiterhin die Verwendung des oben beschriebenen Systems zur optischen Stimulation einer Nervenzelle. Dabei kann vorgesehen sein, dass das System zum Bereitstellen eines optischen Signales *in vitro* und/oder *ex vivo* verwendet wird, oder anders formuliert, dass die Nervenzelle außerhalb eines lebenden biologischen Organismus mittels des Systems optisch stimuliert wird. Alternativ kann das System *in vivo* verwendet werden.

Die Erfindung betrifft weiterhin ein Verfahren zum Herstellen eines Systems zum Bereitstellen eines optischen Signales, und bevorzugt zum Herstellen des oben beschriebenen Systems, umfassend die Schritte
a) Bereitstellen eines Energiewandlers, wobei der Energiewandler wenigstens zwei Materialien umfasst und als magnetoelektrischer Wandler dazu ausgestaltet ist, auf Basis eines magnetischen Wechselfeldes elektrische Energie zu erzeugen,
b) Aufbringen einer Lichtquelle auf dem Energiewandler, durch vakuumbasierte Beschichtungstechnik, derart dass die Lichtquelle eine erste als Elektrode fungierende Oberfläche des Energiewandlers elektrisch leitend kontaktiert, und derart dass die Lichtquelle nicht über eine Kabelverbindung mit dem Energiewandler elektrisch kontaktiert ist.

In anderen Worten dient also der Energiewandler als Substrat für die Lichtquelle, die direkt auf dem Energiewandler aufgebracht wird. Bevorzugt wird die Lichtquelle mittels vakuumbasierter Beschichtungstechnik auf der ersten als Elektrode fungierenden Oberfläche des Energiewandlers abgeschieden, derart dass die Lichtquelle berührend auf der ersten Oberfläche aufliegt. Derart lässt sich besonders einfach ein miniaturisiertes System herstellen. Es kann aber auch vorgesehen sein, dass die erste Oberfläche des Energiewandlers teilweise mit einer Isolation beschichtet wird und die Lichtquelle auf der Isolation aufgebracht wird, derart dass zwischen der Lichtquelle und der ersten Oberfläche die Isolation ist und zugleich beispielweise an einem Rande die Lichtquelle die erste Oberfläche elektrisch leitend kontaktiert.

Im Hinblick auf den oben beschriebenen Schichtaufbau des Energiewandlers ist bevorzugt vorgesehen, dass die Lichtquelle auf der ersten Schicht und besonders bevorzugt auf der Schicht mit magnetostriktivem Material des Energiewandlers abgeschieden wird. In anderen Worten handelt es sich bei der ersten als Elektrode fungierenden Oberfläche also bevorzugt um eine Schicht mit magnetostriktivem Material des Energiewandlers.

Weiter bevorzugt ist vorgesehen, dass der Schritt a) Bereitstellen des Energiewandlers die folgenden Schritte umfasst:
- Bereitstellen zweier Schichten, wobei eine der zwei Schichten als Schicht mit piezoelektrischem Material und eine andere der zwei Schichten als Schicht mit magnetostriktivem Material ausgestaltet ist, und
- Verbinden der zwei Schichten mittels einer Verbindungsschicht bevorzugt aus Epoxy, wobei die Verbindungsschicht mittels Rotationsbeschichtung auf einer der zwei Schichten aufgebracht wird.

Derart kann auf einfache Weise der magnetoelektrische Energiewandler bereitgestellt werden, der im Weiteren dann als Substrat zum Aufbringen der Lichtquelle verwendet wird.

Weiter bevorzugt umfasst der Schritt b) Aufbringen der Lichtquelle auf dem Energiewandler, durch vakuumbasierte Beschichtungstechnik, derart dass die Lichtquelle die erste als Elektrode fungierende Oberfläche des Energiewandlers elektrisch leitend kontaktiert ein Aufdampfen von einzelnen Schichten von wenigstens zwei OLEDs unter Verwendung von Abschattungsmasken. Besonders bevorzugt werden die zwei OLEDs derart aufgedampft, dass sie übereinadergestapelt sind.

Weiter bevorzugt umfasst Schritt b) die Schritte:
- Abscheiden einer Isolationsschicht auf dem Energiewandler, derart dass die Isolationsschicht direkt die erste Oberfläche und/oder Schicht berührt,
- Abscheiden einer Schicht elektrisch leitenden Materials auf der Isolationsschicht mittels Atomlagenabscheidung,
- teilweise Entfernen der Schicht elektrisch leitenden Materials und der Isolationsschicht mittels chemischen und/oder physikalischen Verfahren derart, dass ein Teil der ersten Oberfläche und/oder Schicht freigelegt wird, und
- Aufbringen der Lichtquelle auf dem freigelegten Teil der ersten Oberfläche und/oder Schicht, derart dass die Lichtquelle die erste Oberfläche und/oder Schicht elektrisch leitend kontaktiert.

Alternativ kann im letzten Schritt vorgesehen sein, dass die Lichtquelle auf der Isolationsschicht aufgebracht wird, derart dass die Lichtquelle den freigelegten Teil der ersten Oberfläche und/oder Schicht elektrisch leitend kontaktiert.

Besonders bevorzugt wird zudem die Schicht elektrisch leitenden Materials derart abgeschieden, dass sie die als weitere Elektrode fungierende zweite Schicht des Energiewandlers elektrisch kontaktiert und die Lichtquelle derart auf dem freigelegten Teil der ersten Schicht aufgebracht, dass die Lichtquelle die erste Schicht elektrisch leitend kontaktiert und über die Schicht elektrisch leitenden Materials elektrisch leitendend mit der zweiten Schicht des Energiewandlers elektrisch kontaktiert ist.

Weiter bevorzugt umfasst das Verfahren zudem den Schritt
- Einkapseln des Energiewandlers und der Lichtquelle in eine Abkapselung, derart dass die Lichtquelle und der Energiewandler in die Abkapselung integriert sind. Besonders bevorzugt wird die Abkapselung mittels Gasphasenabscheidung auf dem Energiewandler und der Lichtquelle aufgebracht.

Nachfolgend wird die Erfindung unter Bezugnahme auf die anliegenden Zeichnungen anhand von bevorzugten Ausführungsbeispielen exemplarisch erläutert, wobei die nachfolgend dargestellten Merkmale sowohl jeweils einzeln als auch in Kombination einen Aspekt der Erfindung darstellen können. Es zeigen:
- Fig. 1: eine schematische Schnittdarstellung eines Systems zum Bereitstellen eines optischen Signales, gemäß einer bevorzugten Ausgestaltung der Erfindung,
- Fig. 2: eine schematische Darstellung eines elektrischen Schaltbildes des Systems aus Figur 1, und
- Fig. 3: eine schematische Darstellung einer weiteren Ausführungsform eines Systems zum Bereitstellen eines optischen Signales, gemäß einer weiteren bevorzugten Ausgestaltung der Erfindung.

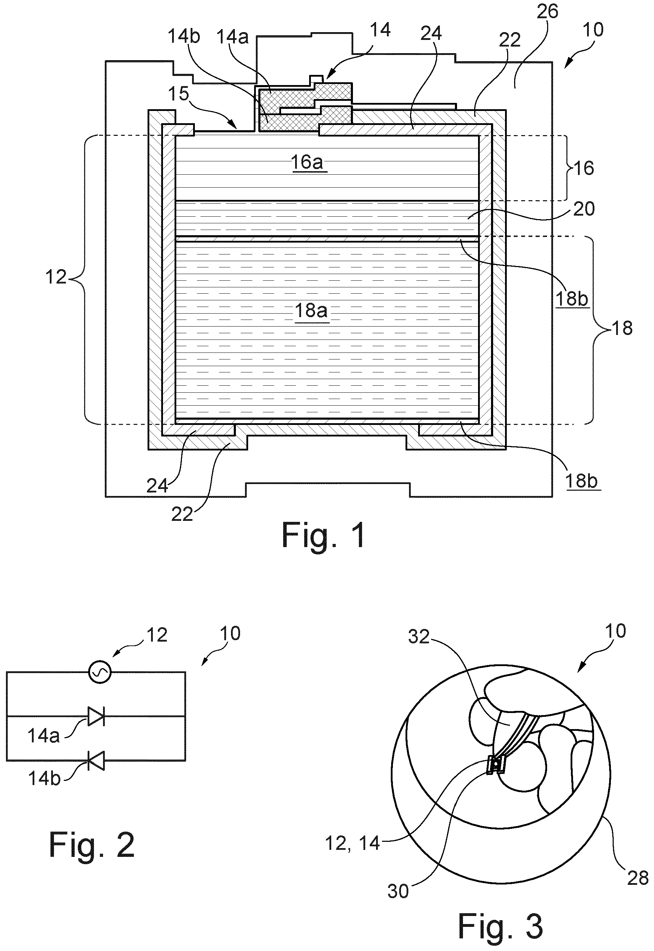

Figur 1 zeigt eine schematische Schnittdarstellung eines Systems 10 zum Bereitstellen eines optischen Signales. Das System 10 umfasst einen Energiewandler 12 und eine Lichtquelle 14, wobei der Energiewandler 12 wenigstens zwei Materialien 16a, 16b umfasst und als magnetoelektrischer Wandler dazu ausgestaltet ist, auf Basis eines magnetischen Wechselfeldes elektrische Energie zu erzeugen. Die Lichtquelle 14 ist auf dem Energiewandler 12 angeordnet und kontaktiert eine als erste Elektrode fungierende Oberfläche 15 des Energiewandlers 12 elektrisch leitend.

Zudem weist der Energiewandler 12 vorliegend einen Schichtaufbau mit wenigstens zwei Schichten 16, 18 auf. Wie vorliegend in Figur 1 zu erkennen, ist die erste Schicht 16 des Energiewandlers 12 als homogene Schicht umfassend das magnetostriktive Material 16a ausgestaltet, wobei als magnetostriktives Material Metglas 2605SA1 (FeSiB Legierung) 16a verwendet wird. Die zweite Schicht 18 des Energiewandlers 12 ist vorliegend als Schicht 18 mit piezoelektrischem Material 18a ausgestaltet. Die zweite Schicht 18 umfasst die homogene Schicht umfassend das piezoelektrische Material 18a Blei-Zirkonat-Titanat 18a, sowie zudem zwei Schichten Nickel 18b, die direkt auf jeweils einer Seite der Blei-Zirkonat-Titanat 18a Schicht aufgebracht sind. Die erste Schicht 16 und die zweite Schicht 18 des Energiewandlers 12 sind über eine Verbindungsschicht 20 - vorliegend aus Epoxy - miteinander verbunden.

Die Lichtquelle 14 ist vorliegend auf der ersten Oberfläche 15 des Energiewandlers 12 aufgebracht, was in vorliegend auch bedeutet, dass die Lichtquelle 14 auf der ersten Schicht 16 des Energiewandlers 12 aufgebracht ist und die erste Schicht 16 elektrisch leitend kontaktiert. Wie zudem in Figur 1 zu erkennen ist, weist das System 10 eine dritte Schicht 22 und eine Isolationsschicht 24 auf. Die Lichtquelle 14 ist über die dritte Schicht 22 elektrisch leitendend mit der zweiten Schicht 18 des Energiewandlers 12 elektrisch kontaktiert, wobei die Isolationsschicht 24 die dritte Schicht 22 von der ersten Schicht 16 elektrisch isoliert.

Weiterhin ist in Figur 1 zu erkennen, dass die Lichtquelle 14 zwei übereinandergestapelte OLEDs 14a, 14b umfasst. Zudem ist die Lichtquelle 14 und der Energiewandler 12 in eine Abkapselung 26 - vorliegend aus Parylen-C - integriert.

Figur 2 zeigt eine schematische Darstellung eines elektrischen Schaltbildes des Systems 10 aus Figur 1. Wie darauf zu erkennen ist, sind die zwei OLEDs 14a, 14b der Lichtquelle 14 antiparallel zueinander geschaltet, so dass jeder der zwei OLEDs 14a, 14b einen eigenen Stromkreis aufweist, und eine der zwei OLEDs 14a, 14b in Sperrrichtung und die andere der zwei OLEDs 14a, 14b in Durchlassrichtung verschaltet ist.

Figur 3 zeigt eine schematische Darstellung einer weiteren Ausführungsform eines Systems 10 zum Bereitstellen eines optischen Signales, gemäß einer weiteren bevorzugten Ausgestaltung der Erfindung. In dieser Ausführungsform umfasst das System 10 zusätzlich zu dem Energiewandler 12 und der Lichtquelle 14 noch eine als Spule 28 ausgestaltete Energiequelle 28. Der Energiewandler 12 und die Lichtquelle 14 sind weiterhin noch auf einen Träger 30 aufgebracht, so dass diese trotz der geringen Abmessungen des Energiewandlers 12 und der Lichtquelle 14 von zusammengenommen 11 mm x 3 mm x 0,16 mm mittels einer Pinzette 32 zum Erzeugen des optischen Signales in die Spule 28 gehalten werden können.

### Bezugszeichenliste

- 10: System
- 12: Energiewandler
- 14: Lichtquelle
- 14a: erste OLED
- 14b: zweite OLED
- 15: erste Oberfläche
- 16: erste Schicht
- 16a: magnetostriktives Material
- 18: zweite Schicht
- 18a: piezoelektrisches Material
- 18b: Nickel
- 20: Verbindungsschicht
- 22: dritte Schicht, leitende Schicht
- 24: Isolationsschicht
- 26: Abkapselung
- 28: Spule
- 30: Träger
- 32: Pinzette

## Patentansprüche

1. System (10) zum Bereitstellen eines optischen Signales umfassend einen Energiewandler (12) und eine Lichtquelle (14),
wobei der Energiewandler (12) wenigstens zwei Materialien (16a, 18a) umfasst und als magnetoelektrischer Wandler dazu ausgestaltet ist, auf Basis eines magnetischen Wechselfeldes elektrische Energie zu erzeugen,
wobei die Lichtquelle (14) auf dem Energiewandler (12) angeordnet ist und eine erste als Elektrode (16) fungierende Oberfläche (15) des Energiewandlers (12) elektrisch leitend kontaktiert, und
wobei die Lichtquelle (14) nicht über eine Kabelverbindung mit dem Energiewandler (12) elektrisch kontaktiert ist.

2. System (10) nach Anspruch 1, wobei eines der wenigstens zwei Materialien (16a, 18a) als piezoelektrisches Material (18a) ausgestaltet ist und das andere der wenigstens zwei Materialien (16a, 18a) als magnetostriktives Material (16a) ausgestaltet ist.

3. System (10) nach einem der vorhergehenden Ansprüche, wobei die Lichtquelle (14) zwei antiparallel geschaltete OLEDs (14a, 14b) umfasst.

4. System (10) nach einem der vorhergehenden Ansprüche, wobei die Lichtquelle (14) zwei übereinander gestapelte OLEDs (14a, 14b) umfasst.

5. System (10) nach einem der vorhergehenden Ansprüche, wobei die Lichtquelle (14) über eine leitende Schicht (22) elektrisch leitendend mit einer zweiten als Elektrode fungierenden Oberfläche des Energiewandlers (12) elektrisch kontaktiert ist und eine Isolationsschicht (24) die leitende Schicht (22) von der ersten Oberfläche (15) elektrisch isoliert.

6. System (10) nach einem der vorhergehenden Ansprüche, wobei der Energiewandler (12) einen Schichtaufbau mit wenigstens zwei Schichten (16, 18) aufweist, wobei das erste Material (16a) und das zweite Material (18a) jeweils als eine Schicht (16, 18) des Energiewandlers (12) ausgebildet ist und wobei die Lichtquelle (14) auf der ersten der zwei Schichten (16, 18) aufgebracht ist und diese elektrisch leitend kontaktiert.

7. System (10) nach dem vorhergehenden Anspruch, wobei die erste Schicht (16) als Schicht mit magnetostriktivem Material (16a) ausgestaltet ist

8. System (10) nach einem der Ansprüche 6 oder 7, wobei zwischen der ersten Schicht (16) und der zweiten Schicht (18) eine Verbindungsschicht (20) angeordnet ist.

9. System (10) nach einem der vorhergehenden Ansprüche, wobei die Lichtquelle (14) und der Energiewandler (12) in eine Abkapselung (26) integriert sind.

10. System (10) nach einem der vorhergehenden Ansprüche, umfassend eine Energiequelle (28), wobei die Lichtquelle (14) und die Energiequelle (28) und/oder der Energiewandler (12) und die Energiequelle (28) nicht über eine elektrische Verbindung miteinander verbunden sind.

11. System (10) nach dem vorhergehenden Anspruch, wobei die Energiequelle (28) als Spule (28) ausgestaltet ist und/oder dazu ausgestaltet ist ein magnetisches Wechselfeld zu erzeugen.

12. Verwendung eines Systems (10) nach einem der Ansprüche 1 bis 11 zur optischen Stimulation einer Nervenzelle.

13. Verfahren zum Herstellen eines Systems (10) zum Bereitstellen eines optischen Signales, umfassend die Schritte
a) Bereitstellen eines Energiewandlers (12), wobei der Energiewandler (12) wenigstens zwei Materialien (16a, 18a) umfasst und als magnetoelektrischer Wandler dazu ausgestaltet ist, auf Basis eines magnetischen Wechselfeldes elektrische Energie zu erzeugen,
b) Aufbringen einer Lichtquelle (14) auf dem Energiewandler (12) durch vakuumbasierte Beschichtungstechnik, derart dass die Lichtquelle (14) eine erste als Elektrode fungierende Oberfläche (15) des Energiewandlers (12) elektrisch leitend kontaktiert, und derart dass die Lichtquelle (14) nicht über eine Kabelverbindung mit dem Energiewandler (12) elektrisch kontaktiert ist.

## Claims

1. A system (10) for providing an optical signal, comprising an energy converter (12) and a light source (14),
wherein the energy converter (12) comprises at least two materials (16a, 18a) and is designed as a magnetoelectric converter for the purpose of generating electrical energy based on an alternating magnetic field,
wherein the light source (14) is arranged on the energy converter (12) and electrically conductively contacts a first surface (15) of the energy converter (12) acting as an electrode (16), and
wherein the light source (14) is not electrically contacted with the energy converter (12) via a cable connection.

2. The system (10) according to claim 1, wherein one of the at least two materials (16a, 18a) is designed as a piezoelectric material (18a), and the other of the at least two materials (16a, 18a) is designed as a magnetostrictive material (16a).

3. The system (10) according to any one of the preceding claims, wherein the light source (14) comprises two OLEDs (14a, 14b) connected in an antiparallel manner.

4. The system (10) according to any one of the preceding claims, wherein the light source (14) comprises two OLEDs (14a, 14b) stacked on top of one another.

5. The system (10) according to any one of the preceding claims, wherein the light source (14) is electrically conductively contacted, via a conducting layer (22), with a second surface of the energy converter (12) acting as an electrode, and an insulating layer (24) electrically insulates the conducting layer (22) from the first surface (15).

6. The system (10) according to any one of the preceding claims, wherein the energy converter (12) has a layered composition comprising at least two layers (16, 18), the first material (16a) and the second material (18a) each being designed as a layer (16, 18) of the energy converter (12), and the light source (14) being applied to and electrically conductively contacting the first of the two layers (16, 18).

7. The system (10) according to the preceding claim, wherein the first layer (16) is designed as a layer comprising magnetostrictive material (16a).

8. The system (10) according to either claim 6 or 7, wherein a connecting layer (20) is arranged between the first layer (16) and the second layer (18).

9. The system (10) according to any one of the preceding claims, wherein the light source (14) and the energy converter (12) are integrated in an encapsulation (26).

10. The system (10) according to any one of the preceding claims, comprising an energy source (28), wherein the light source (14) and the energy source (28) and/or the energy converter (12) and the energy source (28) are not connected to one another via an electrical connection.

11. The system (10) according to the preceding claim, wherein the energy source (28) is designed as a coil (28) and/or is designed to generate an alternating magnetic field.

12. Use of a system (10) according to any one of claims 1 to 11 for the optical stimulation of a nerve cell.

13. A method for producing a system (10) for providing an optical signal, comprising the following steps:
a) providing an energy converter (12), wherein the energy converter (12) comprises at least two materials (16a, 18a) and is designed as a magnetoelectric converter for the purpose of generating electrical energy based on an alternating magnetic field; and
b) applying a light source (14) to the energy converter (12) using a vacuum-based coating technique such that the light source (14) electrically conductively contacts a first surface (15) of the energy converter (12) acting as an electrode and such that the light source (14) is not electrically contacted with the energy converter (12) via a cable connection.

## Revendications

1. Système (10) destiné à fournir un signal optique, comprenant un convertisseur d'énergie (12) et une source lumineuse (14),
dans lequel le convertisseur d'énergie (12) comprend au moins deux matériaux (16a, 18a) et est conçu en tant que convertisseur magnéto-électrique à des fins de génération d'énergie électrique en se basant sur un champ magnétique alternatif,
dans lequel la source lumineuse (14) est agencée sur le convertisseur d'énergie (12) et est en contact de manière électriquement conductrice avec une première surface (15) du convertisseur d'énergie (12) agissant en tant qu'électrode (16), et
dans lequel la source lumineuse (14) n'est pas en contact électrique avec le convertisseur d'énergie (12) par l'intermédiaire d'une connexion par câble.

2. Système (10) selon la revendication 1, dans lequel l'un des au moins deux matériaux (16a, 18a) est conçu en tant que matériau piézoélectrique (18a), et l'autre des au moins deux matériaux (16a, 18a) est conçu en tant que matériau magnétostricteur (16a).

3. Système (10) selon l'une quelconque des revendications précédentes, dans lequel la source lumineuse (14) comprend deux OLED (14a, 14b) connectées de manière antiparallèle.

4. Système (10) selon l'une quelconque des revendications précédentes, dans lequel la source lumineuse (14) comprend deux OLED (14a, 14b) empilées au sommet l'une de l'autre.

5. Système (10) selon l'une quelconque des revendications précédentes, dans lequel la source lumineuse (14) est en contact de manière électriquement conductrice, par l'intermédiaire d'une couche conductrice (22), avec une seconde surface du convertisseur d'énergie (12) agissant en tant qu'électrode, et une couche isolante (24) isole électriquement la couche conductrice (22) de la première surface (15).

6. Système (10) selon l'une quelconque des revendications précédentes, dans lequel le convertisseur d'énergie (12) a une composition en couches comprenant au moins deux couches (16, 18), le premier matériau (16a) et le second matériau (18a) étant chacun conçus en tant que couche (16, 18) du convertisseur d'énergie (12), et la source lumineuse (14) étant appliquée à et en contact électriquement conducteur avec la première des deux couches (16, 18).

7. Système (10) selon la revendication précédente, dans lequel la première couche (16) est conçue en tant que couche comprenant un matériau magnétostricteur (16a).

8. Système (10) selon soit la revendication 6 soit la revendication 7, dans lequel une couche de connexion (20) est agencée entre la première couche (16) et la deuxième couche (18).

9. Système (10) selon l'une quelconque des revendications précédentes, dans lequel la source lumineuse (14) et le convertisseur d'énergie (12) sont intégrés dans une encapsulation (26).

10. Système (10) selon l'une quelconque des revendications précédentes, comprenant une source d'énergie (28), dans lequel la source lumineuse (14) et la source d'énergie (28) et/ou le convertisseur d'énergie (12) et la source d'énergie (28) ne sont pas connectés l'un à l'autre par l'intermédiaire d'une connexion électrique.

11. Système (10) selon la revendication précédente, dans lequel la source d'énergie (28) est conçue en tant que bobine (28) et/ou est conçue pour générer un champ magnétique alternatif.

12. Utilisation d'un système (10) selon l'une quelconque des revendications 1 à 11 pour la stimulation optique d'une cellule nerveuse.

13. Procédé de production d'un système (10) destiné à fournir un signal optique, comprenant les étapes suivantes :
a) fournir un convertisseur d'énergie (12), dans lequel le convertisseur d'énergie (12) comprend au moins deux matériaux (16a, 18a) et est conçu en tant que convertisseur magnéto-électrique à des fins de génération d'énergie électrique en se basant sur un champ magnétique alternatif ; et
b) appliquer une source lumineuse (14) au convertisseur d'énergie (12) en utilisant une technique de revêtement à base de vide de telle sorte que la source lumineuse (14) entre en contact de manière électriquement conductrice avec une première surface (15) du convertisseur d'énergie (12) agissant en tant qu'électrode et de telle sorte que la source lumineuse (14) n'est pas en contact électrique avec le convertisseur d'énergie (12) par l'intermédiaire d'une connexion par câble.
